(19) Europäisches Patentamt
European Patent Office
Office européen des brevets

(11) Veröffentlichungsnummer: **0 259 871**
**A1**

# (12) EUROPÄISCHE PATENTANMELDUNG

(21) Anmeldenummer: **87113229.6**

(22) Anmeldetag: **10.09.87**

(51) Int. Cl.[4]: **C07D 403/04** , C07D 413/04 , C07D 417/04 , A61K 31/40

(30) Priorität: **12.09.86 DE 3631085**

(43) Veröffentlichungstag der Anmeldung: **16.03.88 Patentblatt 88/11**

(84) Benannte Vertragsstaaten: **AT BE CH DE ES FR GB GR IT LI LU NL SE**

(71) Anmelder: **Boehringer Mannheim GmbH**
**Sandhofer Strasse 116**
**D-6800 Mannheim 31(DE)**

(72) Erfinder: **Von der Saal, Wolfgang, Dr. rer. nat.**
**Neuer Burgweg 3**
**D-6940 Weinheim(DE)**
Erfinder: **Friebe, Walter-Gunar, Dr. rer. nat.**
**Sophienstrasse 8**
**D-6800 Mannheim 1(DE)**
Erfinder: **Mertens, Alfred, Dr. rer. nat.**
**Beethovenstrasse 20**
**D-6905 Schriesheim(DE)**
Erfinder: **Müllier-Beckmann, Dr. med. vet.**
**Hochgewanne 46**
**D-6718 Grünstadt(DE)**
Erfinder: **Kling, Lothar, Dr.**
**Kranichweg 37**
**D-6800 Mannheim - 31(DE)**

## (54) Heterocyclisch substituierte Indolinone, Verfahren zu ihrer Herstellung, Arzneimittel sowie Zwischenprodukte.

(57) Heterocyclisch substituierte Indolinone der allgemeinen Formel I

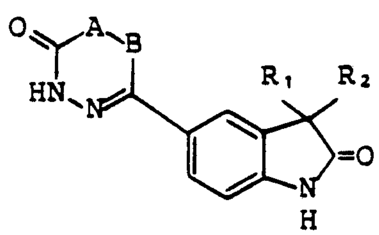

(I),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind, ein Lacton bilden,

EP 0 259 871 A1

A-B eine Gruppe

$$-CH_2-\overset{R_3}{\overset{|}{C}}H-, \quad -CH=\overset{R_3}{\overset{|}{C}}-,$$

$-CH_2-NH-$, $-NH-CH_2-$, $-CH_2-O-$, $-O-CH_2-$, $-CH_2-S-$, $-S-CH_2-$darstellt, wobei $R_3$ ein Wasserstoffatom, eine Alkylgruppe oder eine Hydroxyalkylgruppe bedeutet, oder in welcher
A eine Bindung darstellt und B eine Methylengruppe $>CR_4R_5$ oder eine Iminogruppe $>N-R_6$ darstellt, wobei $R_4$, $R_5$ und $R_6$ Wasserstoffatome oder Alkylgruppen bedeuten,

deren Tautomere und deren physiologisch vertraeglichen Salze anorganischer und organischer Saeuren, Verfahren zu ihrer Herstellung, diese Verbindungen enthaltende Arzneimittel und die Verwendung zur Behandlung von Herz-und Kreislauferkrankungen sowie Zwischenprodukte.

## Heterocyclisch substituierte Indolinone, Verfahren zu ihrer Herstellung, Arzneimittel sowie Zwischenprodukte

Die vorliegende Erfindung betrifft neue heterocyclisch substituierte Indolinone der allgemeinen Formel I,

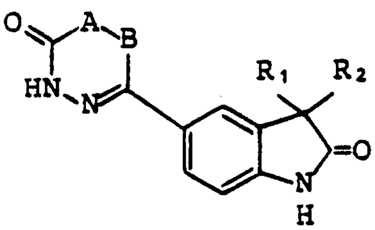

(I),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind ein Lacton bilden,

A-B eine Gruppe

$$-CH_2-\overset{R_3}{CH}-, \quad -CH=\overset{R_3}{C}-,$$

$-CH_2-NH-$, $-NH-CH_2-$, $-CH_2-O-$, $-O-CH_2-$, $-CH_2-S-$, $-S-CH_2-$darstellt, wobei $R_3$ ein Wasserstoffatom, eine Alkylgruppe oder eine Hydroxyalkylgruppe bedeutet, oder in welcher
A eine Bindung darstellt und B eine Methylengruppe $>CR_4R_5$ oder eine Iminogruppe $>N-R_6$ darstellt, wobei $R_4$, $R_5$ und $R_6$ Wasserstoffatome oder Alkylgruppen bedeuten,

deren Tautomere und deren physiologisch vertraeglichen Salze anorganischer und organischer Saeuren, Verfahren zu ihrer Herstellung, diese Verbindungen enthaltende Arzneimittel, sowie Zwischenprodukte.

Die Verbindungen der allgemeinen Formel I besitzen mindestens ein asymmetrisches C-Atom. Gegenstand der Erfindung sind auch die optisch aktiven Formen und die racemischen Gemische dieser Verbindungen.

Die positiv inotrope Wirkung von Verbindungen der allgemeinen Formel I, in der $R_1$ die angegebenen Bedeutungen besitzt und $R_2$ Wasserstoff oder eine Methylgruppe bedeutet, oder in der $R_1$ und $R_2$ zusammen einen Cyclopropanring bilden, und A-B die Gruppe

$$-CH_2-\overset{R_3}{CH}- \text{ oder } -CH=\overset{R_3}{C}-$$

bedeutet, ist aufgrund der Europaeischen Offenlegungsschriften 155 798, 161 918 und 178 876 bereits bekannt. Ueberraschend fanden wir nun, daß Verbindungen der allgemeinen Formel I, in der $R_2$ und A-B die angegebenen Bedeutungen besitzen, ebenfalls eine hervorragende Herzkraft-steigernde Wirkung aufweisen. Darueberhinaus wirken Verbindungen der allgemeinen Formel I blutdrucksenkend und/oder verbessern die Mikrozirkulation und beeinflussen die Thrombozytenfunktion.

Bedeutet $R_1$ eine Alkylgruppe oder Hydroxyalkylgruppe, so sind darunter geradkettige oder verzweigte Alkylketten mit 1-6 Kohlenstoffatomen bevorzugt, insbesondere Methyl, Ethyl oder Propyl und Hydroxymethyl.

Bedeutet $R_2$ eine Alkyloxycarbonylgruppe, eine Hydroxyalkylgruppe oder eine Alkylcarbonyloxyalkylgruppe, so besitzen die geradkettigen oder verzweigten Alkylreste bevorzugt 1-6 C-Atome. Bevorzugte Reste sind die Methoxycarbonyl-, Ethoxycarbonyl-, i-Propyloxycarbonyl-, t.-Butyloxycarbonylgruppe, die Hydroxymethyl-oder die Methylcarboxymethylgruppe.

Bilden $R_1$ und $R_2$ zusammen einen Lactonring, so sind dabei bevorzugt Ringe mit 4, 5 oder 6 Gliedern, insbesondere der γ-Butyrolactonring.

Bilden A-B eine der oben genannten Gruppen, in denen $R_3$ eine Alkylgruppe oder eine Hydroxyalkylgruppe bedeutet, so sind bevorzugt Alkylgruppen mit 1-3 Kohlenstoffatomen, insbesondere Methyl.

Bedeutet A eine Bindung und B eine Gruppe $>CR_4R_5$ oder $>NR_6$ wobei $R_4$-$R_6$ Alkylgruppen darstellen, so sind Alkylgruppen mit 1-3 C-Atomen bevorzugt, insbesondere Methyl.

Besonders bevorzugt sind Verbindungen der allgemeinen Formel I, in der

$R_1$ eine Methyl-, Ethyl-oder Propylgruppe bedeutet,

$R_2$ eine Methoxycarbonyl-, Ethoxycarbonyl-, i-Propyloxycarbonyl-, t.-Butyloxycarbonylgruppe, eine Hydroxymethyl-oder eine Methylcarboxymethylgruppe darstellt,

$R_1$ und $R_2$ zusammen mit dem C-Atom, an das sie gebunden sind, einen γ-Butyrolactonring darstellt,

A-B die Gruppen $-CH_2-CH_2-$, $-CH=CH-$, $-CH_2-\underset{\displaystyle CH_3}{CH}-$,

$-CH_2-\underset{\displaystyle CH_2OH}{CH}-$ , $-CH=\underset{\displaystyle CH_3}{C}-$ , $-O-CH_2-$, $-HN-CH_2-$

darstellt, oder

A eine Bindung und B die Gruppe $-C(CH_3)_2-$bedeutet.

Die Verbindungen der allgemeinen Formel I lassen sich nach an sich bekannten Verfahren herstellen. Besonders vorteilhaft sind jedoch die in Schema a-e gezeigten Verfahren.

Schema a

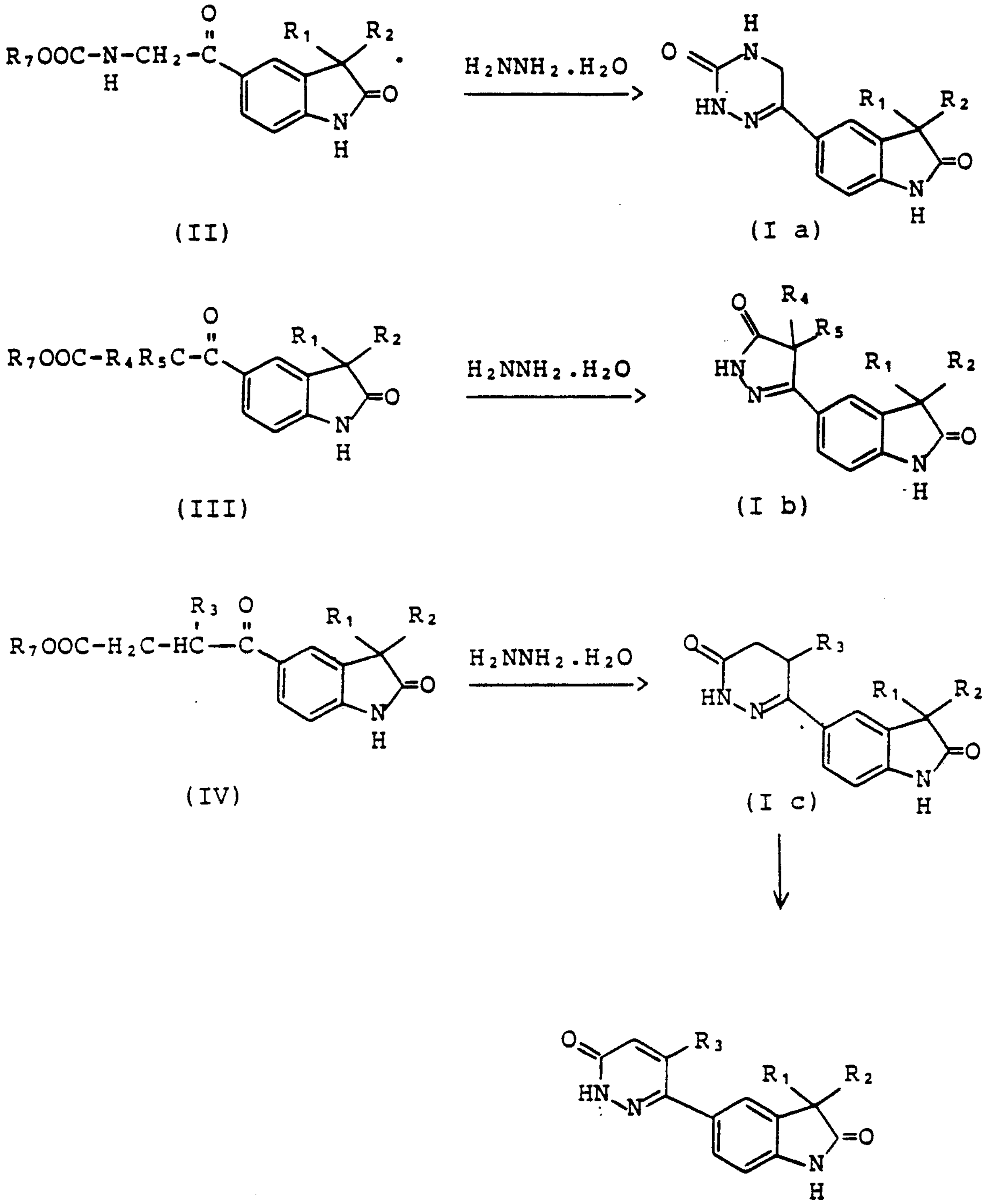


(II) (I a)

(III) (I b)

(IV) (I c)

(I d)

Wie in Schema a gezeigt, lassen sich ein Teil der Verbindungen der allgemeinen Formel I durch Umsetzung der Ketocarbonsaeurederivate II-IV mit Hydrazinhydrat herstellen. Darunter fallen diejenigen Verbindungen der allgemeinen Formel I, in denen $R_1$ und $R_2$ die angegebenen Bedeutungen besitzen und A-B entweder die Gruppe $-NH-CH_2-$ist (Formel I a), oder die Gruppe

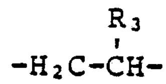

ist (Formel I c), oder in der A eine Bindung und B die Gruppe $>CR_4R_5$ ist (Formel I b), wobei $R_3$, $R_4$ und $R_5$ die oben angegebenen Bedeutungen besitzen. In den Verbindungen der allgemeinen Formeln II-IV haben $R_1$, $R_2$, $R_3$, $R_5$ die oben angegebenen Bedeutungen, $R_7$ bedeutet ein Wasserstoffatom, eine Niederalkylkette wie Methyl-oder Ethyl-, oder eine Phenylgruppe.

Die Umsetzungen der Verbindungen II zu I a, von III zu I b und von IV zu I c erfolgt vorzugsweise in einem Loesungsmittel wie Ethanol, Isopropanol oder in einer Mischung wie Isopropanol/Wasser in Gegenwart von 1-5 mol Hydrazinhydrat, vorzugsweise mit 1-2 mol Hydrazinhydrat, bei Temperaturen zwischen Raumtemperatur und dem Siedepunkt des Loesungsmittels oder Loesungsmittelgemisches.

Verbindungen der allgemeinen Formel I, in denen $R_1$, $R_2$ die angegebenen Bedeutungen besitzen, A-B die Gruppe $-CH_2-CHR_3-$bedeutet (Formel I c), lassen sich anschließend gewuenschtenfalls oxidieren zu Verbindungen der allgemeinen Formel I, in denen A-B die Gruppe $-CH=CR_3-$bedeutet (Formel I d).

Dies gelingt durch Bromierung/Dehydrobromierung, oder durch edelmetallkatalysierte Dehydrierung oder durch Oxidation mit Braunstein oder m-Nitrobenzolsulfonsaeure.
(vgl. J. Med. Chem. 17, 273 (1974)).

Schema b

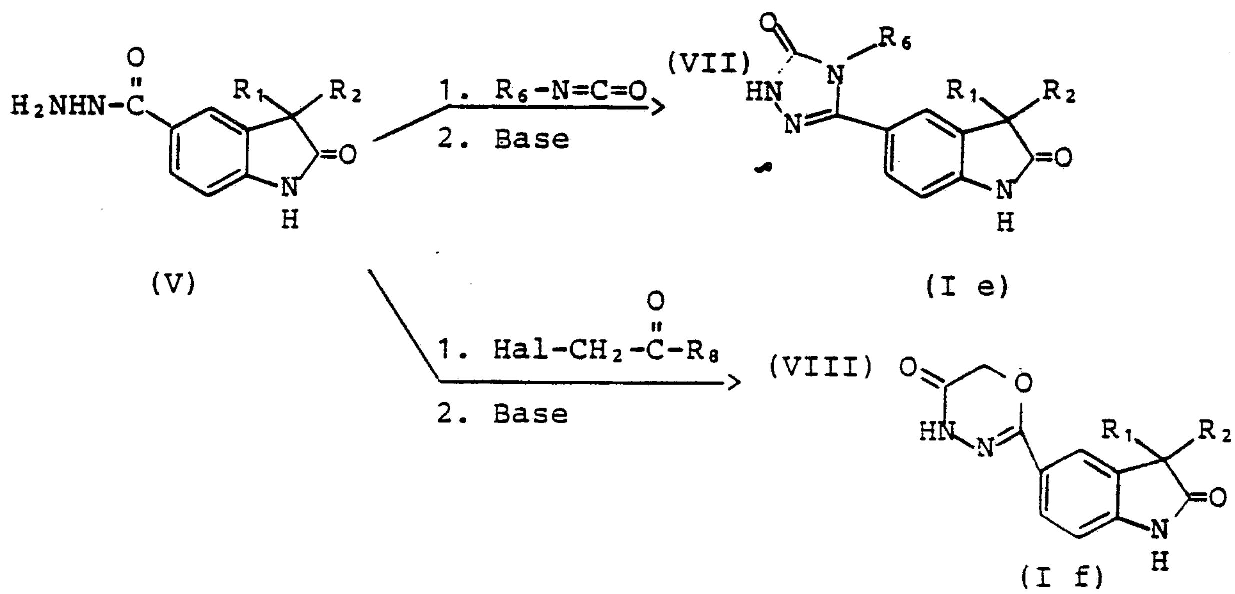


(V) (VII) (I e) (VIII) (I f)

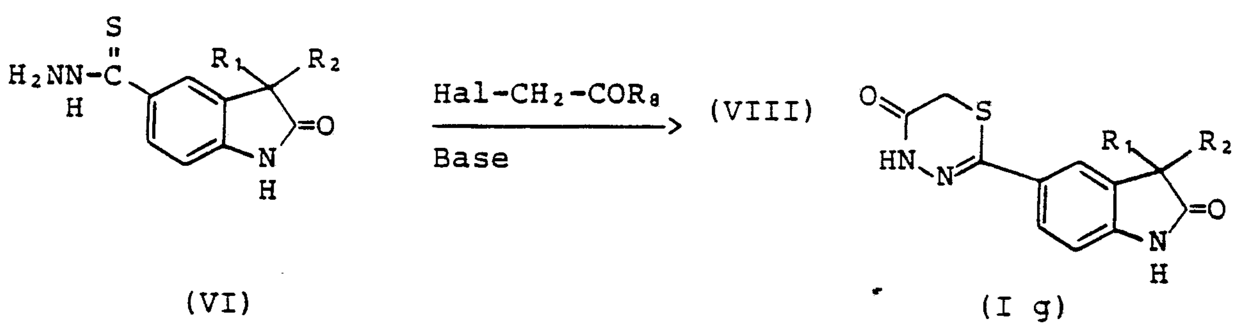


(VI) (VIII) (I g)

Wie in Schema b gezeigt, lassen sich ein Teil der Verbindungen der allgemeinen Formel I durch Umsetzung der Hydrazide der allgemeinen Formeln V oder VI mit den Isocyanaten VII oder den Halogencarbonsaeuren VIII in Gegenwart von Basen herstellen. Dies betrifft solche Verbindungen der allgemeinen Formel I, in der $R_1$, $R_2$ die oben angegebenen Bedeutungen haben und A-B entweder die Gruppe $-CH_2-O-$ (Formel I f), oder die Gruppe $-CH_2-S-$(Formel I g) bedeutet, oder in der A eine Bindung und B eine Iminogruppe $>NR_6$ (Formel I e) darstellt. In den Isocyanaten der allgemeinen Formel VII hat $R_8$ die oben angegebene Bedeutung. In den Halogenessigsaeuren der allgemeinen Formel VIII bedeutet Hal ein Halogenatom wie Chlor, Brom oder Jod, vorzugsweise jedoch Chlor, und $R_8$ bedeutet Halogen wie vorzugsweise Chlor, oder eine Alkoxygruppe, vorzugsweise Methoxy-oder Ethoxy.

Die Synthesen der Verbindungen der allgemeinen Formeln I e und I f fuehrt man vorzugsweise in zwei Schritten durch.

Im ersten Schritt setzt man ein Hydrazid der allgemeinen Formel V entweder mit einem Isocyanat der allgemeinen Formel VII oder mit einer Halogencarbonsaeure der allgemeinen Formel VIII um, und zwar bevorzugt in inerten Loesungsmitteln wie Toluol oder Dioxan und in Gegenwart einer Base wie Kaliumcarbonat. In beiden Faellen erhaelt man als Zwischenprodukte Diacylhydrazine, die man nun in einem dipolar aprotischen Loesungsmittel, z.B. Dimethylformamid, mit einer Base wie Natriumhydrid, oder mit einem Alkalimetallcarbonat in Aceton reagieren laeßt. Vorzugsweise fuehrt man diesen Schritt bei erhoehter Temperatur, etwa bei 100°C, durch, und erhaelt unmittelbar die Verbindungen der allgemeinen Formeln I e bzw. I f.

Die Umsetzung der Thiohydrazide der allgemeinen Formel VI zu Verbindungen der allgemeinen Formel I g fuehrt man vorzugsweise in einem Schritt durch. Dabei verwendet man vorzugsweise ein aktiviertes Derivat einer Halogencarbonsaeure der allgemeinen Formel VIII, z.B. einen Methyl-oder Ethylester. Die Umsetzung fuehrt man bevorzugt in waessriger Loesung bei Raumtemperatur in Gegenwart einer Base wie Natriumhydroxid durch.

Schema c

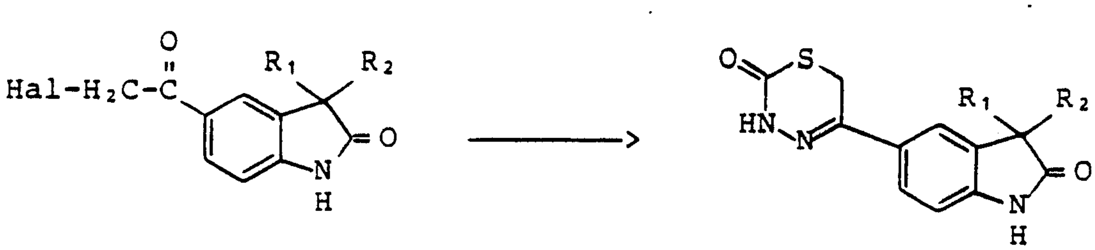

(IX) (I h)

Verbindungen der allgemeinen Formel I, in der $R_1$, $R_2$ die oben angegebenen Bedeutungen hat und A-B die Gruppe $-S-CH_2-$bedeutet (Formel I h) stellt man vorzugsweise durch Umsetzung von Verbindungen der allgemeinen Formel IX mit Ethylthiocarbazat oder Methylthiocarbazat her. Die Reaktion fuehrt man in einem organischen Loesungsmittel, vorzugsweise Acetonitril oder Ethanol bei erhoehter Temperatur, vorzugsweise beim Siedepunkt des Loesungsmittels durch.

Schema d

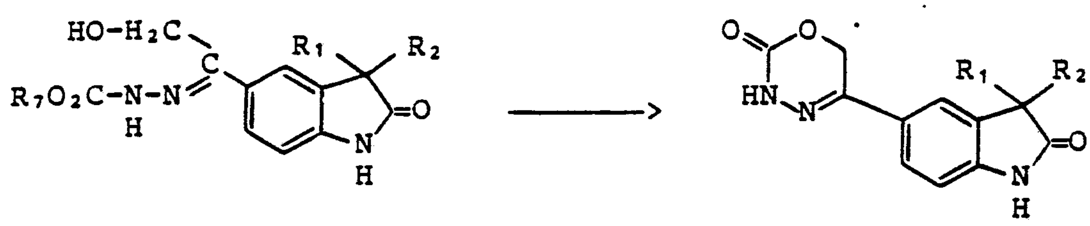

(X) (I i)

Verbindungen der allgemeinen Formel I, in der $R_1$, $R_2$ die angegebenen Bedeutungen haben und A-B die Gruppe $-O-CH_2-$bedeutet (Formel I i) stellt man durch Cyclisierung von Hydrazonen der allgemeinen Formel X, in der $R_1$, $R_2$ und $R_7$ die angegebenen Bedeutungen besitzen, her.

Vorzugsweise fuehrt man die Cyclisierung in Gegenwart einer Base, wie z.B. Natriummethylat, in einem Loesungsmittel wie Ethanol bei Raumtemperatur aus.

Schema e

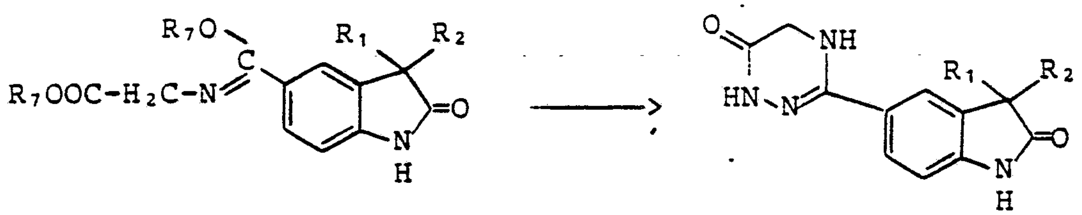

(XI) (I k)

Verbindungen der allgemeinen Formel I, in der $R_1$, $R_2$ die angegebenen Bedeutungen besitzen und A-B die Gruppe $-CH_2-NH-$bedeutet (Formel I k), stellt man aus den Iminoethern der allgemeinen Formel XI her, in der $R_1$, $R_2$ und $R_7$ die angegebenen Bedeutungen besitzen, durch Umsetzung mit Hydrazinhydrat. Diese Reaktion fuehrt man vorzugsweise in einem Loesungsmittel wie Ethanol bei Temperaturen bis zum Siedepunkt des Loesungsmittels durch.

Die Verbindungen der allgemeinen Formel J koennen auch nachtraeglich in andere Verbindungen der allgemeinen Formel I umgewandelt werden.

Dies trifft zu fuer die Reduktion von Verbindungen der allgemeinen Formel I, in der $R_1$ und A-B die angegebenen Bedeutungen haben und $R_2$ eine Estergruppe darstellt, zu solchen Verbindungen der allgemeinen Formel I, in der $R_2$ die Hydroxymethylgruppe darstellt. Diese Reduktion fuehrt man vorzugsweise durch Lithium-Aluminiumhydrid in einem inerten Loesungsmittel durch.

Dies trifft weiterhin zu fuer die Veresterung von Verbindungen der allgemeinen Formel I, in der $R_1$ und A-B die angegebenen Bedeutungen haben und $R_2$ eine Hydroxyalkylgruppe darstellt, zu Verbindungen der allgemeinen Formel I, in der $R_2$ eine Alkylcarbonyloxyalkylgruppe darstellt.

Dies trifft weiterhin zu fuer die Veresterung von Verbindungen der allgemeinen Formel I, in der $R_1$ und A-B die angegebenen Bedeutungen haben und $R_2$ eine Carboxylgruppe darstellt,zu solchen Verbindungen der allgemeinen Formel I, indenen$R_2$ eine Alkoxycarbonylgruppe bedeutet. Die genannten Veresterungen werden nach literaturbekannten Methoden durchgefuehrt.

Dies trifft weiterhin zu fuer die Hydrolyse und Alkoholyse von Verbindungen der allgemeinen Formel I, in der $R_1$ und A-B die oben angegebenen Bedeutungen haben und $R_2$ eine Alkoxycarbonylgruppe bedeutet zu solchen Verbindungen der allgemeinen Formel I, in der $R_2$ eine Carboxylgruppe bedeutet (Verseifung) oder eine Alkoxycarbonylgruppe (Umesterung) bedeutet. Die Verseifung und Umesterungen werden nach literaturbekannten Methoden durchgefuehrt.

Die fuer die Reaktionenin Schema a-e benoetigten Ausgangsverbindungen II, III, IV, V, VI, IX, X und XI sind ebenfalls neu und Gegenstand dieser Erfindung.

Ihre Darstellung erfolgt ueber eine Friedel-Crafts-Reaktion zwischen Saeurehalogeniden oder -anhydriden und den Oxindolen der allgemeinen Formel XII,

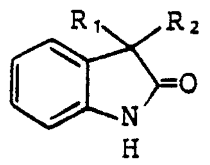

(XII),

wobei $R_1$, $R_2$ die oben angegebenen Bedeutungen besitzen.

Die Verbindungen der allgemeinen Formel XII sind literaturbekannt oder lassen sich nach literaturbekannten Verfahren herstellen (vgl. R.M. Acheson et al., J. Chem. Soc. Perkin I 1979, 595).

Durch Umsetzung der Verbindungen der allgemeinen Formel XII mit den Esterchloriden

$R_7OOC\text{-}R_4R_5C\text{-}COCl$

erhaelt man die Verbindungen der allgemeinen Formel III, durch Umsetzung mit den Esterchloriden

$R_7OOC\text{-}CH_2\text{-}CHR_3\text{-}COCl$

erhaelt man die Verbindungen der allgemeinen Formel IV, durch Umsetzung mit Halogenessigsaeurechloriden erhaelt man die Verbindungen der allgemeinen Formel IX, und durch Umsetzung mit Acetylchlorid die Verbindungen der allgemeinen Formel XIII.

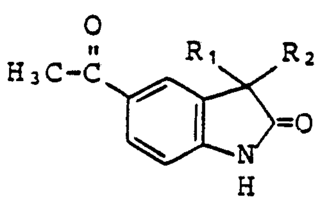

(XIII)

Diese Friedel-Crafts-Reaktionen fuehrt man in einem Loesungsmittel wie Kohlendisulfid, Methylenchlorid, Dichlorethan oder Nitrobenzol in Gegenwart eines Ueberschusses einer Lewissaeure wie Aluminiumchlorid oder Aluminiumbromid bei Temperaturen zwischen 0°C und 150°C, vorzugsweise beim Siedepunkt des Loesungsmittels, oder in Gegenwart eines großen Ueberschusses (bis 10 mol) Aluminiumchlorid in Dimethylformamid bei Temperaturen zwischen 0°C und 150°C durch.

Die als Ausgangsmaterial benoetigten Verbindungen der allgemeinen Formel II sind in zwei Schritten aus den Verbindungen der allgemeinen Formel IX zugaenglich.

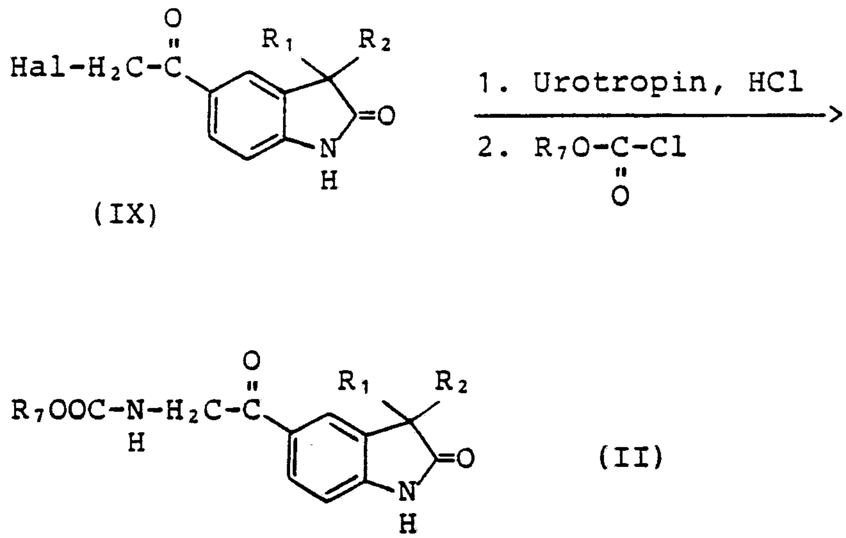


(IX)

(II)

Durch Umsetzung der Verbindungen IX mit Urotropin und anschließender saurer Hydrolyse erhaelt man ein α-Aminoketon, welches man mit Chlorameisensaeureestern am N-Atom acyliert und so die Verbindungen der allgemeinen Formel II erhaelt (vgl. Europaeische Offenlegungsschrift 80 296).

Die restlichen als Ausgangsmaterial benoetigten Verbindungen sind aus den Verbindungen der allgemeinen Formel XIII zugaenglich.

So erhaelt man die Verbindungen der allgemeinen Formel V, indem man die Verbindungen der allgemeinen Formel XIII der Haloform-Reaktion unterwirft, wobei durch Hypochlorit oder Hypobromit die Acetylgruppe zur Saeure oxidiert wird, und anschließend mit

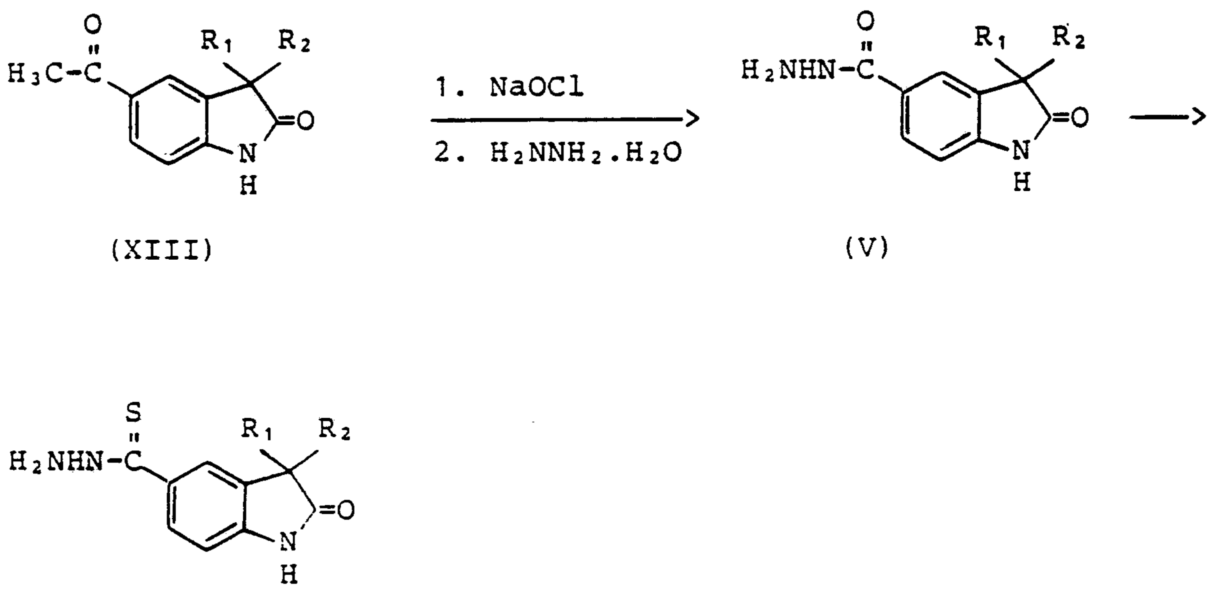


(XIII)

(V)

(VI)

Hydrazinhydrat umsetzt. Aus diesen Verbindungen wiederum erhaelt man durch Umsetzung mit einem Schwefeluebertragenden Reagens die ebenfalls als Ausgangsmaterial benoetigten Verbindungen der allgemeinen Formel VI.

Die durch Hypochlorit-Oxidation der Verbindungen XIII erhaltene Carbonsaeure dient auch der Darstellung der Iminoether der allgemeinen Formel XI. Dies geschieht durch Alkylierung der Amide mittels Trialkyloxoniumsalzen (vgl. Perst, Oxonium Ions in Organic Chemistry , S. 128-137, Verlag Chemie, 1971).

Oxidiert man dagegen die Verbindungen der allgemeinen Formel XIII mit einem milderen Oxidationsmittel, bevorzugt mit Jodbenzol-Diacetat, so lassen sich α-Hydroxyketone erhalten, die man Alkylcarbazaten zu den als Ausgangsverbindungen benoetigten Verbindungen der allgemeinen Formel X umsetzt (vgl. Europaeische Offenlegungsschrift 80 296).

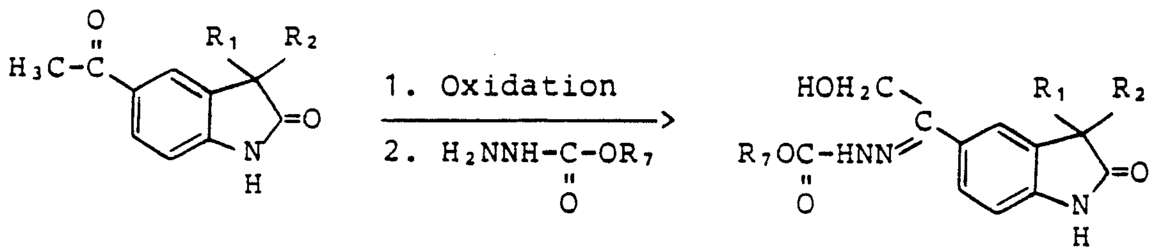


(XIII) (X)

Ferner koennen die erhaltenen Verbindungen der allgemeinen Formel I anschließend gewuenschtenfalls in ihre physiologisch vertraeglichen Saeureadditionssalze mit anorganischen oder organischen Saeuren ueberfuehrt werden. Als Saeuren kommen hierfuer beispielsweise Salzsaeure, Bromwasserstoffsaeure, Schwefelsaeure, Phosphorsaeure, Fumarsaeure, Bernsteinsaeure, Weinsaeure, Zitronensaeure, Milchsaeure, Maleinsaeure oder Methansulfonsaeure in Betracht.

Wie bereits eingangs erwaehnt weisen die neuen Verbindungen der allgemeinen Formel I, deren Tautomere und deren physiologisch vertraegliche Saeureadditionssalze bei einer langen Wirkungsdauer ueberlegene pharmakologsche Eigenschaften auf, insbesondere eine blutdrucksenkende und/oder positivinotrope Wirkung und/oder beeinflussen die Thrombozytenfunktion und verbessern die Mikrozirkulation.

Zur Herstellung von Arzneimitteln werden die Substanzen der allgemeinen Formel I in an sich bekannter Weise mit geeigneten pharmazeutischen Traegersubstanzen, Aroma-, Geschmacks-und Farbstoffen gemischt und beispielsweise als Tabletten oder Dragees ausgeformt oder unter Zugabe entsprechender Hilfsstoffe in Wasser oder Oel, wie z.B. Olivenoel, suspendiert oder geloest.

Die erfindungsgemaeßen neuen Substanzen der allgemeinen Formel I und ihre Salze koennen in fluessiger oder fester Form enteral oder parenteral appliziert werden. Als Injektionsmedium kommt vorzugsweise Wasser zur Anwendung, welches die bei Injektionsloesungen ueblichen Zusaetze wie Stabilisierungsmittel, Loesungsvermittler oder Puffer enthaelt.

Derartige Zusaetze sind z.B. Tartrat-und Citratpuffer, Ethanol, Komplexbildner (wie Ethylendiamintetraessigsaeure und deren nicht toxische Salze) und hochmolekulare Polymere (wie fluessiges Polyethylenoxid) zur Viskositaetsregulierung. Feste Traegerstoffe sind z.B. Staerke, Lactose, Mannit, Methylcellulose, Talkum, hochdisperse Kieselgelsaueren, hochmolekulare Fettsaeuren (wie Stearinsauere), Gelatine, Agar-Agar, Calciumphosphat, Magnesiumstearat, tierische und pflanzliche Fette und feste hochmolekulare Polymere (wie Polyethylenglykole). Fuer orale Applikation geeignete Zubereitungen koennen gewuenschtenfalls Geschmacks-und Sueßstoffe enthalten.

Die erfindungsgemaeßen Verbindungen werden ueblicherweise in Mengen von 10-500 mg pro Tag bezogen auf 75 kg Koerpergewicht appliziert. Bevorzugt ist es, 2-3 mal pro Tag 1-2 Tabletten mit einem Wirkstoffgehalt von 5-200 mg zu verabreichen. Die Tabletten koennen retardiert sein, wodurch nur noch 1 mal pro Tag 1-2 Tabletten mit 10-500 mg Wirkstoff gegeben werden muß. Der Wirkstoff kann auch durch Injektion 1-8 mal pro Tag bzw. durch Dauerinfusion gegeben werden, wobei Mengen von 5-200 mg/Tag normalerweise ausreichen.

Bevorzugt im Sinne der vorliegenden Erfindung sind außer den in den Beispielen genannten Verbindungen die folgenden und deren Tautomere:

5-(2,3,4,5-Tetrahydro-3-oxo-pyridazin-6-yl)-3-tert.butyloxy-carbonyl-3-methyl-1H-indolin-2-on

5-(2,3,4,5-Tetrahydro-3-oxo-pyridazin-6-yl)-3-hydroxymethyl-3-methyl-1H-indolin-2-on

5-(2,3,4,5-Tetrahydro-3-oxo-pyridazin-6-yl)-3-methylcarbonyl-oxymethyl-3-methyl-1H-indolin-2-on

5′-(2,3,4,5-Tetrahydro-3-oxo-pyridazin-6-yl)spiro[tetrahydro-furan-2-on-3,3′-indolin-2′-on]

5-(5-Methyl-2,3,4,5-tetrahydro-3-oxo-pyridazin-6-yl)-3-methyl-3-ethoxycarbonyl-1H-indolin-2-on

5-(5-Hydroxymethyl-2,3,4,5-tetrahydro-3-oxo-pyridazin-6-yl)-3-methyl-3-ethoxycarbonyl-1H-indolin-2-on

5-(2,3-Dihydro-2-oxo-6H-1,3,4-oxodiazin-5-yl)-3-ethoxycarbonyl-3-methyl-1H-indolin-2-on

5-(2,3,4,5-Tetrahydro-3-oxo-1,2,4-triazin-6-yl)-3-ethoxycarbonyl-3-methyl-1H-indolin-2-on

5-(3,4-Dihydro-2H-4,4-dimethyl-3-oxo-pyrazol-5-yl)-3-ethoxycarbonyl-3-methyl-1H-indolin-2-on

Bevorzugte Vorstufen im Sinne der Erfindung (allgemeine Formeln II, III, IV, V, VI, IX, X und XI) sind außer den in den Beispielen genannten die folgenden:

5-(Ethoxycarbonylamino-acetyl)-3-ethoxycarbonyl-3-methyl-indolin-2-on

5-(2-Ethoxycarbonyl-2-methyl-propionyl)-3-ethoxycarbonyl-3-methyl-indolin-2-on

5-(3-Ethoxycarbonyl-2-hydroxymethyl-propionyl)-3-ethoxycarbonyl-3-methyl-indolin-2-on

5-Hydrazinocarbonyl-3-ethoxycarbonyl-3-methyl-indolin-2-on

5-Hydrazinothiocarbonyl-3-ethoxycarbonyl-3-methyl-indolin-2-on

5-(Hydroxyacetyl-ethoxycarbonylhydrazon)-3-ethoxycarbonyl-3-methyl-indolin-2-on

5-(3-Methoxycarbonyl-propionyl)-3-methoxycarbonyl-3-methyl-indolin-2-on

5-(3-Methoxycarbonyl-propionyl)-3-ethoxycarbonyl-3-propyl-indolin-2-on

5-(3-Methoxycarbonyl-propionyl)-3-isopropyloxycarbonyl-3-methyl-indolin-2-on

5-(3-Methoxycarbonyl-propionyl)-3-tert.butyloxycarbonyl-3-methyl-indolin-2-on

5-Chlormethylcarbonyl-3-ethoxycarbonyl-3-methyl-1H-indolin-2-on

5-(Ethoxycarbonyl-ethoxycarbonylmethylimin)-3-ethoxycarbonyl-3-methyl-1H-indolin-2-on

Beispiel 1

5-(2,3,4,5-Tetrahydro-3-oxo-pyridazin-6-yl)-3-ethoxycarbonyl-3-methyl-1H-indolin-2-on

Zu 2.4 g (7.2 mmol) 3-Ethoxycarbonyl-3-methyl-5-(3-methoxycarbonyl-propionyl)-1H-indolin-2-on in 50mlEthanol gibt man 0.34ml (7.2 mmol) Hydrazinhydrat und erhitzt über Nacht zum Rückfluß. Nach Abkühlen wird filtriert. Man isoliert 1.4 g Titelverbindung (62 % d. Th.) vom Schmp. 274-276°C.

Das Ausgangsmaterial stellt man auf folgende Weise her:

3-Ethoxycarbonyl-3-methyl-5-(3-methoxycarbonyl-propionyl)-1H-indolin-2-on

Zu einer Mischung aus 12.1 g (85 mmol) Aluminiumchlorid und 40 ml Kohlendisulfid tropft man bei 15-20°C 3.4 ml (27 mmol) 3-Methoxycarbonyl-propionylchlorid und anschließend eine Lösung von 5.0 g (23 mmol) 3-Ethoxycarbonyl-3-methyloxindol in 10 ml Kohlendisulfid und 5ml Dichlormethan. Man rührt 20 h bei Raumtemperatur, dekantiert die Lösung ab, zersetzt den harzigen Rückstand mit Eis und extrahiert mit Dichlormethan.

Nach Chromatographie an Kieselgel (Elutionsmittel Dichlormethan/Methanol 99:1) erhält man neben 2.4 g nicht umgesetzten 3-Ethoxycarbonyl-3-methyl-oxindol 2.9 g Titelverbindung (38 % d. Th.) vom Schmp. 107-109°C.

Beispiel 2

In analoger Weise wie in Beispiel 1 beschrieben erhält man 5-(2,3,4,5-Tetrahydro-3-oxo-pyridazin-6-yl)-3-methoxycarbonyl-3-methyl-1H-indolin-2-on in 58proz. Ausbeute mit dem Schmp. 272-273°C (aus Methanol).

Das Ausgangsmaterial 3-Methoxycarbonyl-3-methyl-5-(3-methoxycarbonyl-propionyl)-1H-indolin-2-on vom Schmp. 148-150°C erhält man analog der Vorstufe von Beispiel 1 aus 3-Methoxycarbonyl-3-methyl-oxindol (Ausbeute 45 % d. Th.).

Beispiel 3

5-(2,3,4,5-Tetrahydro-3-oxo-pyridazin-6-yl)-3-isopropyloxycarbonyl-3-methyl-1H-indolin-2-on

Zu einer Lösung von 1.0 g Natrium in 60ml Isopropanol gibt man 3.2 g (10 mmol) 5-(2,3,4,5-Tetrahydro-3-oxo-pyridazin-6-yl)-3-ethoxycarbonyl-3-methyl-1H-indolin-2-on (Verbindung des Beispiels 1), erhitzt 1 h zum Rückfluß, stellt durch Zugabe von ethanolischer Chlorwasserstofflösung sauer, engt im Vakuum ein, nimmt den Rückstand in Wasser und Dichlormethan auf, filtriert, engt die organische Phase ein, vereinigt mit dem Filterrückstand und chromatographiert an Kieselgel (Elutionsmittel 1,1,1-Trichlorethan/Isopropanol 9:1). Man isoliert 1.8 g Titelverbindung (55 % d. Th.) vom Schmp. 290-291°C (aus Diethylether).

Beispiel 4

5-(2,3-Dihydro-3-oxo-pyridazin-6-yl)-3-ethoxycarbonyl-3-methyl-1H-indolin-2-on

Eine Lösung von 2.0 g (6.3 mmol) 5-(2,3,4,5-Tetrahydro-3-oxo-pyridazin-6-yl)-3-ethoxycarbonyl-3-methyl-1H-indolin-2-on (Verbindung des Beispiels 1) in 300 ml Dioxan wird mit 34 g Braunstein 48 h bei 90°C gerührt. Man filtriert, wäscht mit Dichlormethan und Methanol nach, engt die Filtrate ein und verreibt mit Ether. Es verbleiben 0.8 g der Titelverbindung (41 % d. Th.) vom Schmp. 289-291°C.

Beispiel 5

5-(2,3,4,5-Tetrahydro-3-oxo-pyridazin-6-yl)-3-propyl-3-ethoxycarbonyl-1H-indolin-2-on

In analoger Weise wie in Beispiel 1 beschrieben erhält man die Titelverbindung in 35proz. Ausbeute mit dem Schmp. 190-192°C (aus Ether).

Das Ausgangsmaterial 3-Ethoxycarbonyl-3-propyl-5-(3-methoxycarbonyl-propionyl)-1H-indolin-2-on kann wie folgt hergestellt werden:

a) Eine Lösung von 48 g (0.17 mol) 2-(2-Nitrophenyl)-malonsäurediethylester in 33 ml 1-Iodpropan wird mit einer Lösung von 5.3 g Natrium in 600 ml Ethanol versetzt, über Nacht zum Rückfluß erhitzt, eingeengt, mit Wasser versetzt und mit Ether extrahiert. Nach Einengen des Extraktes erhält man 48 g (88 % d.Th.) 2-(2-Nitrophenyl)-2-propyl-malonsäurediethylester als Rohprodukt.

b) 48 g der unter a) erhaltenen Verbindung werden in 800 ml Ethanol gelöst und über 12 g Raney-Nickel bei 4 bar Wasserstoffdruck hydriert. Man filtriert, engt ein und chromatographiert an Kieselgel. Mit 1,1,1-Trichlorethan eluiert man 20.1 g 3-Ethoxycarbonyl-3-propyl-1H-indolin-2-on (55 % d.Th.) vom Schmp. 91-93°C (aus Ligroin).

c) Analog der Vorstufe von Beispiel 1 erhält man 3-Ethoxycarbonyl-3-propyl-5-(3-methoxycarbonyl-propionyl)-1H-indolin-2-on als Öl in 84proz. Ausbeute aus dem unter b) beschriebenen Zwischenprodukt.

## Ansprüche

1. Heterocyclisch substituierte Indolinone der allgemeinen Formel I

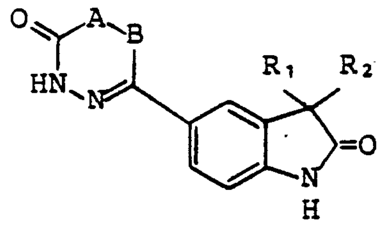

(I),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind ein Lacton bilden,

A-B eine Gruppe

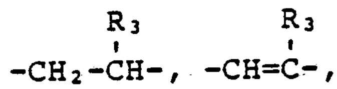

$-CH_2-NH-$, $-NH-CH_2-$, $-CH_2-O-$, $-O-CH_2-$, $-CH_2-S-$, $-S-CH_2-$darstellt, wobei $R_3$ ein Wasserstoffatom, eine Alkylgruppe oder eine Hydroxyalkylgruppe bedeutet, oder in welcher A eine Bindung darstellt und B eine Methylengruppe $>CR_4R_5$ oder eine Iminogruppe $>N-R_6$ darstellt, wobei $R_4$, $R_5$ und $R_6$ Wasserstoffatome oder Alkylgruppen bedeuten,

deren Tautomere und deren physiologisch vertraeglichen Salze anorganischer und organischer Saeuren.

2. Verfahren zur Herstellung von Verbindungen der allgemeinen Formel I

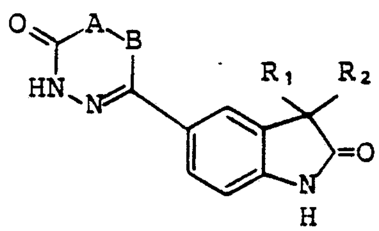

(I),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind ein Lacton bilden,

A-B eine Gruppe

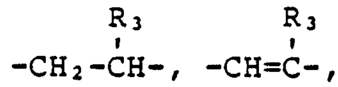

$-CH_2-NH-$, $-NH-CH_2-$, $-CH_2-O-$, $-O-CH_2-$, $-CH_2-S-$, $-S-CH_2-$darstellt, wobei $R_3$ ein Wasserstoffatom, eine Alkylgruppe oder eine Hydroxyalkylgruppe bedeutet, oder in welcher
A eine Bindung darstellt und B eine Methylengruppe $>CR_4R_5$ oder eine Iminogruppe $>N-R_6$ darstellt, wobei $R_4$, $R_5$ und $R_6$ Wasserstoffatome oder Alkylgruppen bedeuten,

deren Tautomere und deren physiologisch vertraeglichen Salze anorganischer und organischer Saeuren, dadurch gekennzeichnet, daß man

a) fuer den Fall, daß die Gruppe A-B eine Gruppe

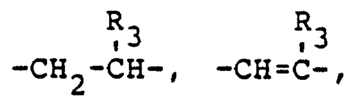

$-NH-CH_2$ oder A eine Bindung und B eine Methylengruppe $>CR_4R_5$ darstellt,

eine Verbindung der Formel XIV

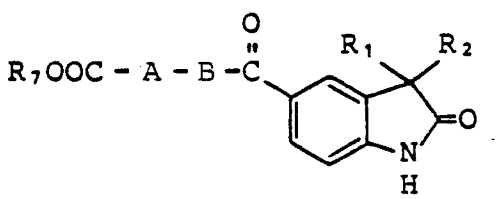 (XIV),

in der

A-B, $R_1$ und $R_2$ die angegebenen Bedeutungen haben und $R_7$ ein Wasserstoffatom, eine Phenylgruppe oder eine niedere Alkylgruppe darstellt,
mit Hydrazin bzw. Hydrazinhydrat gegebenenfalls in Gegenwart eines Alkohols umsetzt,

oder

b) fuer den Fall, daß die Gruppe A-B eine Gruppe $-CH_2-O-$oder $-CH_2-S-$darstellt,

eine Verbindung der Formel XV

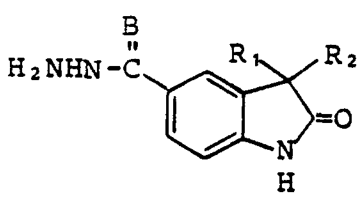 (XV),

in der

B Sauerstoff oder Schwefel darstellt und $R_1$ und $R_2$ die angegebenen Bedeutungen haben

mit einer Verbindung

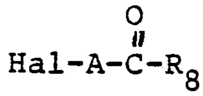

in der

A eine $-CH_2$-Gruppe, Hal ein Halogenatom und $R_8$ ein Halogenatom oder eine nieder Alkoxygruppe darstellen, in Gegenwart einer Base umsetzt

oder

c) fuer den Fall, daß A eine Bindung und B eine Iminogruppe $>N-R_6$ darstellen,

eine Verbindung der Formel V

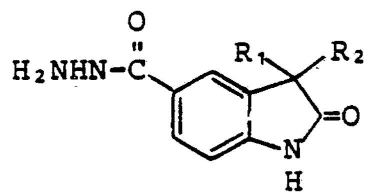

(V),

in der

$R_1$ und $R_2$ die angegebenen Bedeutungen haben, mit einem Isocyanaten der Formel

$R_6-N=C=O$

in der

$R_6$ die angegebene Bedeutung hat, in Gegenwart einer Base umsetzt,

oder

d) fuer den Fall, daß die Gruppe A-B die Gruppe $-S-CH_2$-darstellt,

eine Verbindung der Formel IX

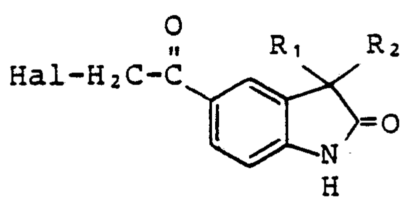

(IX),

in der

$R_1$ und $R_2$ die angegebenen Bedeutungen haben und Hal ein Halogenatom darstellt, mit Alkylthiocarbazat in Gegenwart eines organischen Loesungsmittels umsetzt,

oder

e) fuer den Fall, daß die Gruppe A-B die Gruppe $-O-CH_2$-darstellt,

eine Verbindung der Formel X

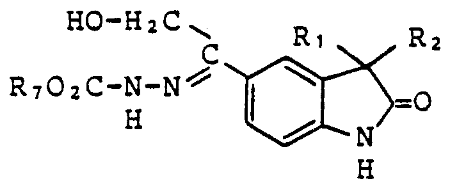

(X),

in der

$R_1$ und $R_2$ die angegebenen Bedeutungen haben und $R_7$ ein Wasserstoffatom, eine Phenylgruppe oder eine niedere Alkylgruppe darstellen,
in Gegenwart einer Base cyclisiert

oder

f) fuer den Fall, daß die Gruppe A-B die Gruppe $-CH_2-NH-$darstellt,

eine Verbindung der Formel XI

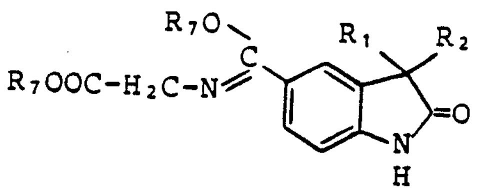

(XI),

in der

$R_1$ und $R_2$ die angegebenen Bedeutungen haben und $R_7$ ein Wasserstoffatom, eine Phenylgruppe oder eine niedere Alkylgruppe darstellen,
mit Hydrazinhydrat in Gegenwart von Alkoholen umsetzt,

worauf man gewuenschtenfalls die erhaltenen Verbindungen der Formel I in andere Verbindungen der Formel I ueberfuehrt und gegebenenfalls die erhaltenen Verbindungen in deren physiologisch vertraegliche Salze ueberfuehrt.

3. Arzneimittel, enthaltend mindestens eine Verbindung gemaeß Anspruch 1 neben ueblichen Traeger- und Hilfsstoffen.

4. Verwendung von Verbindungen gemaeß Anspruch 1 zur Behandlung von Herz-und Kreislauferkrankungen.

5. Verbindungen der Formel XIV

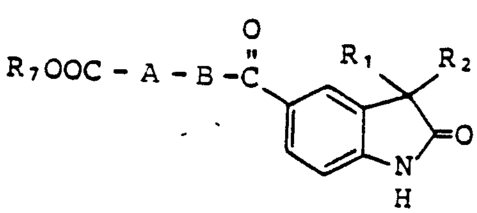

(XIV),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind, ein Lacton bilden,

$R_7$ Wasserstoff, Phenyl oder eine niedere Alkylgruppe und

A-B eine Gruppe

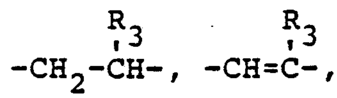

-NH-$CH_2$ oder A eine Bindung und B eine Methylengruppe $CR_4R_5$ darstellt, wobei

$R_3$ ein Wasserstoffatom, eine Alkylgruppe oder eine Hydroxyalkylgruppe und

$R_4$ und $R_5$ Wasserstoffatome oder Alkylgruppen bedeuten,

bedeuten.

6. Verbindungen der Formel XV

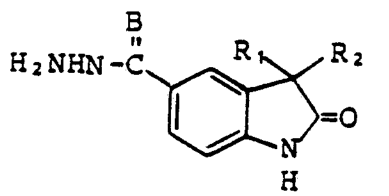

(XV),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind, ein Lacton bilden und

B Sauerstoff oder Schwefel

bedeuten.

7. Verbindungen der Formel IX

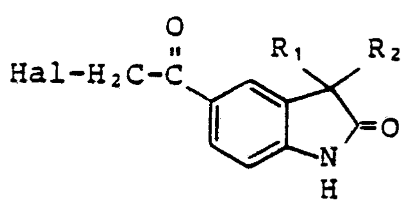

(IX),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind, ein Lacton bilden und

Hal ein Halogenatom

bedeuten.

8. Verbindungen der Formel X

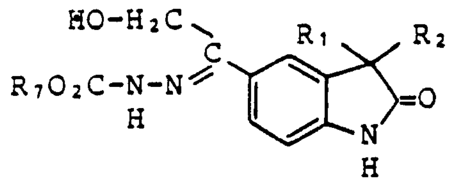

(X),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind, ein Lacton bilden und

$R_7$ Wasserstoff, Phenyl oder eine niedere Alkylgruppe

bedeuten.

9. Verbindungen der Formel XI

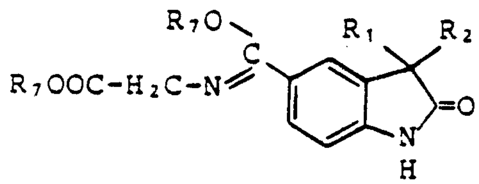

(XI),

in welcher

$R_1$ ein Wasserstoffatom, eine Alkylgruppe oder eine Hydroxyalkylgruppe darstellt,

$R_2$ eine Carboxylgruppe, eine Alkoxycarbonylgruppe, eine Hydroxyalkylgruppe oder eine Alkylcarbonyloxyalkylgruppe bedeutet, oder

$R_1$ und $R_2$ zusammen mit dem C-Atom, an das sie gebunden sind, ein Lacton bilden und

$R_7$ Wasserstoff, Phenyl oder eine niedere Alkylgruppe

bedeuten.

10. Verbindungen der Formel I gemäß Anspruch 1, in der

$R_1$ eine Methyl-, Ethyl-oder Propylgruppe bedeutet,

$R_2$ eine Methoxycarbonyl-, Ethoxycarbonyl-, i-Propyloxycarbonyl-, t.-Butyloxycarbonylgruppe, eine Hydroxymethyl-oder eine Methylcarboxymethylgruppe darstellt,

$R_1$ und $R_2$ zusammen mit dem C-Atom, an das sie gebunden sind, einen $\gamma$-Butyrolactonrung darstellt,

A-B die Gruppen $-CH_2-CH_2-$, $-CH=CH-$, $-CH_2-CH-$,
$CH_3$

$-CH_2-CH-$ , $-CH=C-$ , $-O-CH_2-$, $-HN-CH_2-$
$CH_2OH$ $CH_3$

darstellt, oder

A eine Bindung und B die Gruppe $-C(CH_3)_2-$bedeutet.

Europäisches Patentamt

# EUROPÄISCHER RECHERCHENBERICHT

Nummer der Anmeldung

EP 87 11 3229

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 161 918 (ELI LILLY CO.)<br>* Insgesamt * | 1-10 | C 07 D 403/04<br>C 07 D 413/04<br>C 07 D 417/04<br>A 61 K 31/40 |
| D,Y | EP-A-0 080 296 (IMPERIAL CHEMICAL INDUSTRIES PLC)<br>* Zusammenfassung * | 1-10 | |
| D,Y | EP-A-0 155 798 (ELI LILLY CO.)<br>* Insgesamt * | 1-4 | |
| Y | GB-A-2 031 404 (YOSHITOMI PHARMACEUTICAL INDUSTRIES LTD)<br>* Seiten 1,2 * | 1-5 | |
| A | EP-A-0 178 876 (ELI LILLY CO.)<br>* Zusammenfassung * | 1-5 | |
| A | CHEMICAL ABSTRACTS, Band 91, 1979, Seite 494, Zusammenfassung Nr. 5240s, Columbus, Ohio, US; & JP-A-79 16 485 (YOSHITOMI PHARMACEUTICAL INDUSTRIES, LTD) 07-02-1979<br>* Zusammenfassung * | 1-5 | **RECHERCHIERTE SACHGEBIETE (Int. Cl.4)** |
| A | EP-A-0 068 310 (BASF)<br>* Zusammenfassung * | 1-5 | C 07 D 403/00<br>C 07 D 413/00<br>C 07 D 417/00 |

Der vorliegende Recherchenbericht wurde für alle Patentansprüche erstellt

| Recherchenort | Abschlußdatum der Recherche | Prüfer |
|---|---|---|
| DEN HAAG | 26-11-1987 | MAISONNEUVE J.A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument